(19) Europäisches Patentamt
European Patent Office
Office européen des brevets

(11) **EP 3 351 166 A1**

(12) **EUROPEAN PATENT APPLICATION**

(43) Date of publication:
**25.07.2018 Bulletin 2018/30**

(51) Int Cl.:
***A61B 5/00*** (2006.01)

(21) Application number: **18151048.8**

(22) Date of filing: **10.01.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **23.01.2017 JP 2017009716**

(71) Applicant: **CANON KABUSHIKI KAISHA Ohta-ku Tokyo 146-8501 (JP)**

(72) Inventor: **SENTOKU, Koichi Ohta-ku, Tokyo 146-8501 (JP)**

(74) Representative: **Naidu, Deepal Swamy Canon Europe Limited 3 The Square Stockley Park Uxbridge, Middlesex UB11 1ET (GB)**

(54) **PHOTOACOUSTIC APPARATUS**

(57)    A photoacoustic apparatus is configured to adjust an irradiation range and a quantity of light to be emitted to a subject according to the size of a field of view.

# FIG.1A

**Description**

FIELD OF THE INVENTION

The present invention relates to a photoacoustic apparatus.

BACKGROUND

Photoacoustic tomography (hereinafter referred to as a PAT) using a combination of optical and ultrasonic waves has been provided as one of the methods for imaging hemoglobin inside blood vessels of a subject (a living body). An apparatus using the PAT (hereinafter referred to as a photoacoustic apparatus) includes at least a light source and a probe.

When a subject surface (e.g. the surface of a living body) is irradiated with pulsed light generated by the light source, the light propagates while diffusing inside the subject. An optical absorber inside the subject absorbs the propagating light and expands as a result. This expansion generates a photoacoustic wave. The probe detects such a photoacoustic wave, and outputs a detection signal based on the detected photoacoustic wave. Such a detection signal may be analyzed to acquire the initial sound pressure distribution arising from the optical absorber inside the subject. In the PAT technique, a sound pressure P of ultrasonic wave to be acquired from an optical absorber inside a subject can be expressed by Equation 1 below.

$$\text{Equation 1:} \quad P = \Gamma \cdot \mu_a \cdot \Phi$$

In Equation 1, P is an initial sound pressure, $\Gamma$ is a Gruneisen coefficient that is an elastic property value, $\mu_a$ is an absorption coefficient of the optical absorber, and $\Phi$ is a quantity of light absorbed by the optical absorber. The Gruneisen coefficient is determined by dividing a product of a volumetric expansion coefficient $\beta$ and a square of sound speed c by a specific heat $C_p$. According to Equation 1, the absorption coefficient can be acquired by considering a quantity of light to reach an optional position with respect to the initial sound pressure in such an optional position. Since an absorption coefficient varies depending on an optical absorber, acquisition of absorption coefficient distribution of a subject helps understanding of distribution of a light absorber in the subject, for example, distribution of blood vessels.

Japanese Patent Application Laid-Open No. 2016-112168 discusses a configuration of a photoacoustic measurement apparatus including a probe group of a plurality of probes arranged on an inner wall of a hemispherical cup.

SUMMARY OF THE INVENTION

The inventor of the present invention has identified a potential issue with conventional photoacoustic apparatuses. Specifically, the inventor has identified that issues may arise because the field of view (FOV) in such photoacoustic apparatuses are preferably changed according to size of the subject.

For example, in a case where an area near a distal interphalangeal joint of one finger is intended to be measured in a small subject using the PAT technique, the FOV may be set wider than necessary and then the area may be irradiated with light. In such a case, signals from a portion other than the subject or signals from a range other than a target range can be generated. As a result, there is a possibility that such signals may become noise that may degrade the accuracy of a measurement result. Japanese Patent Application Laid-Open No. 2016-112168 described above does not discuss a change in size of the field of view. However, the present inventor has appreciated that when the size of the field of view is changed, an appropriate apparatus configuration or setting is necessary.

The present invention is directed to a photoacoustic apparatus that has an appropriate apparatus configuration according to a change in a field of view.

According to an aspect of the present invention, there is provided a photoacoustic apparatus as specified in claims 1 to 12.

Further features of the present invention will become apparent from the following description of exemplary embodiments with reference to the attached drawings.

BRIEF DESCRIPTION OF THE DRAWINGS

Figs. 1A and 1B are diagrams illustrating an overall configuration of a photoacoustic apparatus according to a first exemplary embodiment.

Figs. 2A and 2B respectively illustrate an enlarged view of one portion of Fig. 1A and an enlarged view of one portion of Fig. 1B. Figs. 2C, 2D, and 2E are diagrams illustrating a change in light energy distribution according to the first exemplary embodiment.

Figs. 3A and 3B are diagrams illustrating a light quantity adjustment unit.

Figs. 4A, 4B, and 4C are diagrams illustrate the configuration of a photoacoustic apparatus and an optical system adjustment unit according to a second exemplary embodiment.

Figs. 5A and 5B are diagrams illustrate the configuration of a photoacoustic apparatus and an optical system adjustment unit according to a third exemplary embodiment.

Figs. 6A and 6B are diagrams illustrate the configuration of a photoacoustic apparatus and an optical system adjustment unit according to a fourth exemplary embodiment.

DESCRIPTION OF THE EMBODIMENTS

Exemplary embodiments of the present invention are hereinafter described. However, the exemplary embodiments are merely examples, and the present invention is not limited thereto.

A photoacoustic apparatus according to an exemplary embodiment includes a light emission unit that emits light to a subject, an ultrasonic wave probe that detects an ultrasonic wave generated from the subject irradiated with the light to output an electric signal, and an information acquisition unit that acquires information about the subject based on at least the electric signal. Thus, it will be appreciated that the emitted light from the light emission unit irradiates the sample, and that the irradiated sample generates an ultrasonic wave based upon (e.g. in response to) the received light. The photoacoustic apparatus also includes an optical system adjustment unit that can adjust an area over which the subject is irradiated, and a light quantity adjustment unit that can adjust intensity of light that irradiates the subject. Moreover, the optical system adjustment unit can change the irradiation range according to the size of a field of view of the photoacoustic apparatus. The light quantity adjustment unit can change a quantity of light to be emitted to the subject according to the size of a field of view of the photoacoustic apparatus. Since the photoacoustic apparatus according to the exemplary embodiment has such a configuration, a suitable quantity of light and a suitable irradiation light can be provided for irradiating the sample according to the size of a field of view. As a result, an appropriate photoacoustic image according to the size of a field of view is acquired. As a result, more accurate results can be acquired.

(Field of View)

The term "field of view (FOV)" to be used in the present exemplary embodiment represents an area in which a photoacoustic image can be acquired at high resolution by a photoacoustic apparatus according to the present exemplary embodiment. For example, a field of view can be an area from a position at which sensitivity for detection of an ultrasonic wave by the photoacoustic apparatus of the present exemplary embodiment becomes the maximum value to a position at which the sensitivity becomes a half of the maximum value. However, the value is not limited to the half of the maximum value. Herein, the field of view may define, for example, an area of the sample over which the detection sensitivity of the ultrasonic wave probe changes from a maximum value to half of the maximum value. For example, the field of view may be a spherical area with a radius that extends from a position at which the sensitivity is the maximum value to a position at which the sensitivity becomes a half of the maximum value.

A photoacoustic image may be formed using an ultrasonic detection element disposed on a spherical shell. In such a case, a field of view according to the present exemplary embodiment may be defined (e.g. set) based on at least a parameter such as size of the detection element, a position at which the detection element is to be disposed, and a characteristic of a reception frequency of the detection element.

Moreover, an ultrasonic probe including a cup-shaped support portion and a plurality of detection elements arranged on the support portion to detect an ultrasonic wave may be used. In such a case, a field of view according to the present exemplary embodiment can be defined (e.g. set) based on at least a radius of a surface of a plurality of the detection element, a radius of the cup-shaped support member, and the maximum value of a reception (e.g. response) frequency of the detection element. Herein, the detection element has a surface that detects an ultrasonic wave and is circular.

Hereinafter, a field of view can also be referred to as an FOV.

(Photoacoustic Apparatus)

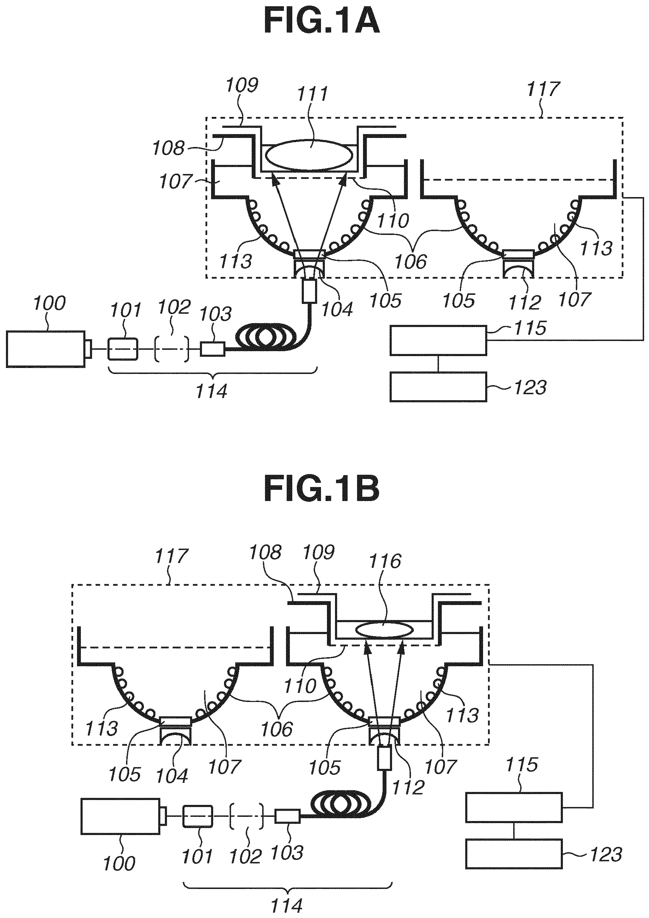

Each of Figs. 1A and 1B is a schematic diagram of a photoacoustic apparatus according to a first exemplary embodiment. The apparatuses illustrated in Figs. 1A and 1B have the same configuration. The apparatuses of Figs. 1A and 1B each have two ultrasonic wave probes (cup-shaped sensors) 106. The ultrasonic wave probes are each arranged to have different FOV sizes and are replaceable. The ultrasonic wave probes are each movable relative to a light emission unit (an illumination optical system) 114. For example, Fig. 1A illustrates a state in which the ultrasonic wave probe 106 having a wide FOV is positioned so as to be attached to the light emission unit 114, whereas, Fig. 1B illustrates a state in which the ultrasonic wave probe 106 having a narrow FOV is attached to the light emission unit. In other words, Fig. 1A illustrates an example case in which photoacoustic measurement is performed with respect to a wide FOV range, whereas Fig. 1B illustrates an example case in which photoacoustic measurement is performed with respect to a narrow FOV range.

In the present exemplary embodiment, it will be appreciated that the arrangement of the photoacoustic apparatus may be changed to provide a wide FOV or a narrow FOV. For example, in a case where a measurement system is shifted from a narrow FOV to a wide FOV, the two cup-shaped sensors (ultrasonic wave probes) 106 attached to an optical system adjustment unit 117 are moved in parallel with respect to an emission end of an optical fiber 103. Each cup-shaped sensor (ultrasonic wave probe) 106 includes a respective lens 104, 112 and glass member 105. Lenses 104 and 112 and the glass member 105 are each peripheral optical systems.

The photoacoustic apparatus may also include an image capturing tab 108 which is arranged to hold a subject 111,116. The image capturing tab 108 includes a mesh member 110 having an opening and a waterproof member 109. In use, a photoacoustic wave propagating from the subject 111, 116 is received by the cup-shaped sensor 106. Each cup shaped sensor includes a plurality of acoustic detectors 113 that are arranged on an inner wall surface that contacts water 107. The photoacoustic apparatus also includes: an optical system adjustment unit 117 which controls the positions of the two cup-shaped sensors 106; a light source unit 100 that generates light; and a light emission unit (an illumination optical system) 114 that receives light from the light source unit 100 and emits light to the subject 111,116. The illumination optical system 114 includes a respective: light quantity adjustment unit 101; light quantity monitor 102; optical fiber 103; lens 104, 112 which is arranged to receive light from the optical fiber 103 and to form the received light into a desired size for illuminating/irradiating the subject 111,116; and, optionally, glass member 105 which is attached to the bottom of a respective cup-shaped sensor 106. A photoacoustic wave signal from the acoustic detector 113 is transmitted to a signal receiving unit 115 via a coaxial cable, for example. The signal receiving unit 115 amplifies the photoacoustic wave signal, and converts the photoacoustic wave signal from an analog signal into a digital signal. Then, the signal receiving unit 115 transmits the photoacoustic wave digital signal to a signal processing unit 123. The signal processing unit 123 performs processing such as integration processing on the photoacoustic wave digital signal to generate subject information.

The FOV of the photoacoustic apparatus can be changed by changing the ultrasonic wave probe. For example, in a case where an ultrasonic wave probe includes a plurality of acoustic wave detectors, the FOV may be changed by changing the position of, or number of, the acoustic wave detectors that are to be used for detecting ultrasonic waves.

As described above, the ultrasonic wave probe and the light emission unit can or cannot be replaced according to the size of the FOV.

Each of the subjects 111 and 116 form an image capturing target and may, for example, be a breast for a breast cancer examination at a breast oncology department or a hand or foot for a blood vessel examination at dermatology or orthopedics department. Each component of a photoacoustic apparatus is described in detail below.

Accordingly, even if an FOV is changed, PAT measurement using the above-described photoacoustic apparatus enables an irradiation range and a quantity of illumination light with respect to a subject to be optimized based on the change in the FOV. Hence, an image having a high signal to noise (S/N) ratio can be acquired.

Hereinafter, each unit of the photoacoustic apparatus according to the present exemplary embodiment is described.

(Light Source Unit)

A light source unit 100 in the present exemplary embodiment emits pulsed light at a wavelength which is absorbed by a specific component of a living body. The living body may comprise a plurality of components, and a portion or all of these components may absorb light at a particular wavelength or range of wavelengths. The wavelength to be used in the present exemplary embodiment is desirably a wavelength at which light propagates into an inside of a subject. Thus, it will be appreciated that the light source unit is arranged to emit light at a wavelength which allows the light to propagate through at least a portion of the subject/sample. Preferably, the light propagates through to an inside of the subject/sample. In this way, it will be appreciated that the light can be considered to penetrate the subject/sample. Preferably, if the subject is a living body for example, the wavelength of the light emitted from the light source unit 100 is 600nm, 1100nm or between 600 nm and 1100 nm. The pulse width is preferably about 10 to 100 nanoseconds so as to efficiently generate a photoacoustic wave. A high-power laser is preferably used as a light source. However, a light emitting diode (LED) or a flash lamp can be used instead of the laser. Moreover, examples of the lasers include various lasers such as a solid-state laser, a gas laser, a dye laser, and a semiconductor laser. Irradiation timing, waveform, and intensity are controlled by a light source control unit. The light source control unit can be integrated with the light source. Moreover, the light source unit 100 can be arranged separately from the photoacoustic apparatus of the present exemplary embodiment.

The light source unit 100 of the present exemplary embodiment can be a light source that can emit light having a plurality of wavelengths. In other words, the light source unit 100 may be a tunable light source unit that can be tuned to output a plurality of wavelengths. Each of these wavelengths may have a spectral width.

(Light Quantity Adjustment Unit)

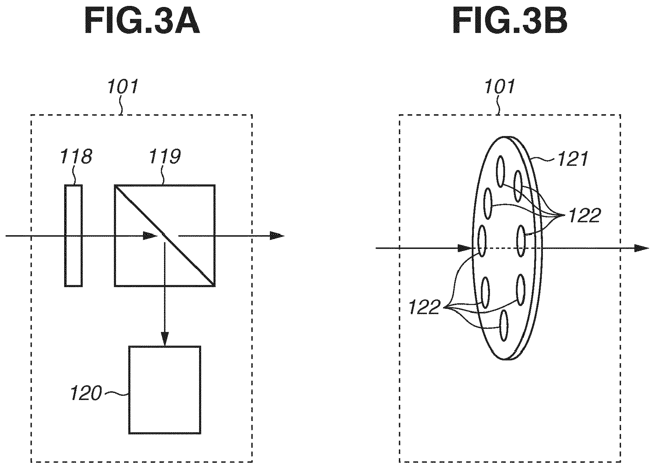

Figs. 3A and 3B illustrate a configuration of the light quantity adjustment unit 101 according to the present exemplary embodiment. A intensity of light is adjusted to increase or decrease a quantity of light energy of illumination light to be emitted to a subject according to the size of an FOV. Thus, it will be appreciated that the light quantity adjustment unit 101 changes the intensity of the light from the light source unit 100. Preferably, the light quantity adjustment unit 101 attenuates the light from the light source unit 100 to output an attenuated light beam. Details of how the light quantity adjustment unit 101 changes the light intensity is described below.

The light quantity adjustment unit 101 illustrated in Fig. 3A includes a $\lambda/2$ wavelength plate 118, a polarization beam splitter 119, and an optical absorption member 120 capable of absorbing light reflected by the polarization beam splitter 119. The light from the light source unit 100 includes linearly polarized light. The light received by the light quantity adjustment unit 101 from the light source unit 100 passes through the $\lambda/2$ wavelength plate 118, and is then split into P-polarized light (which is transmitted through the beam split surface) and S-polarized light (which is reflected by the beam split surface) by the beam split surface of the polarization beam splitter 119. Herein, rotation of the $\lambda/2$ wavelength plate 118 about an optical axis changes the polarization direction of the linearly polarized light from the light source unit 100. In this way, the $\lambda/2$ wavelength plate 118 is used to change the polarization of the light that is incident on the polarization beam splitter 119, so that the ratio of the transmitted P-polarized light to the reflected S-polarized light is changed. Consequently, it will be appreciated that the amount of S-polarized light and P-polarized light that is emitted by the polarization beam splitter 119 can be optionally set. Thus, if a light energy quantity of illumination light (intensity of light) to be emitted to a subject is intended to be increased, the $\lambda/2$ wavelength plate 118 may be set in a rotation angle position so that light of a P-polarized component is increased. On the other hand, if a light energy quantity of the illumination light to be emitted to the subject is intended to be decreased, a rotation angle of the $\lambda/2$ wavelength plate 118 may be set so that light of an S-polarized component is increased. The optical absorption member 120 absorbs S-polarized component light that does not move toward the subject, and prevents stray light.

Fig. 3B illustrates a light quantity adjustment unit which includes an optical component that is different from that illustrated in Fig. 3A. Specifically, the light quantity adjustment unit of Fig. 3B includes a plurality of optical filters (neutral density (ND) filters) 122 which are attached to a disk 121. The optical filters each have a different transmittance value. The disk 121 is rotated to select an ND filter having a desired transmittance, so that a light quantity of illumination light is adjusted. For example, the disk 121 may be rotated so that received light from the light source unit 100 passes through, and is attenuated by, a given optical filter. In this way, the light quantity adjustment unit provides an attenuated light output, which has a smaller intensity/power than the light emitted directly from light source unit 100.

(Optical System Adjustment Unit)

An optical system adjustment unit in the present exemplary embodiment is capable of adjusting an irradiation range of illumination light to be emitted to a subject. That is, the optical system adjustment unit in the present exemplary embodiment is arranged to adjust the area over which a subject is illuminated/irradiated. One cup-shaped sensor and one optical system are integrated (hereinafter referred to as an optical system integrated sensor or an optical system integrated probe), and the photoacoustic apparatus includes a plurality of optical system integrated sensors. Focal distances of the optical systems differ from each other, and an optical system integrated sensor having a desired irradiation range is selected according to the size of an FOV. Each of Figs. 1A and 1B illustrates a photoacoustic apparatus which includes two optical system integrated sensors. Lenses 104 and 112 have different focal distances and are each attached to a respective cup-shaped sensor 106. Depending on which optical system integrated sensor is arranged to receive light from the light emission unit, the light from the light emission unit may be emitted to a subject via lens 104 or lens 112. Each of the photoacoustic apparatuses is arranged such that the lens through which the light from the light emission unit passes may be changed according to size of an FOV. Each of the optical system integrated sensors are attached to the movable optical system adjustment unit 117, as mentioned previously. Moreover, an optical axial position of at least one of the lenses in the movable optical system adjustment unit 117 can be moved to change an irradiation range which changes according to size of an FOV.

(Information Acquisition Unit)

An information acquisition unit in the present exemplary embodiment includes a support portion (a casing) (corresponding to the cup-shaped sensor 106 illustrated in Fig. 1A), and a plurality of detection elements (the acoustic detectors 113 illustrated in Fig. 1A) arranged on the support portion.

(Support Portion)

A support portion in the present exemplary embodiment can be a casing which has a curved surface (a cup-shaped casing) according to the below description. Alternatively, the support portion may be a hand-held type support portion such as an hand-held ultrasonic probe.

Herein, a subject may be a breast. In such a case, a burden on the subject is smaller if the breast is held by a support portion having a curved surface since pressure applied to the breast is smaller than a case in which a breast is held by a plate-shaped (i.e. flat-shaped) holding unit. Thus, a cup-shaped support portion including a plurality of detection elements is preferably used. The present exemplary embodiment is described using an example case in which a hemispherical support portion is used. However, a support portion may be in a substantially hemispherical shape, a truncated cone shape, a truncated pyramid shape, or a semi-cylindrical shape other than the hemispherical shape. Moreover, the substantially hemispherical shape has an angle x that can be smaller than 90 degrees or larger than 90 degrees. The angle x is made by a line connecting the center of the sphere to an apex of the sphere and a line connecting the center of the sphere to an edge of the sphere. If the angle x is 90 degrees, it is hemispherical shape. Optionally, if a plurality of cup-shaped sensors are attached to one photoacoustic apparatus - as similar to the present exemplary embodiment - the size of the cup-shaped sensors may differ from each other.

(Detection Element)

A detection element in the present exemplary embodiment detects photoacoustic waves generated on a surface of a living body and an inside of the living body, and outputs a detection signal based on the detected photoacoustic waves. The photoacoustic waves are generated by the living body based on the pulsed light that irradiates the living body. The detection element converts a photoacoustic wave into an electric signal. Any detection element such as a detection element using a piezoelectric phenomenon, a detection element using resonance of light, and a detection element using a change in electrostatic capacitance can be used, so long as it can detect a photoacoustic wave. An example of the detection element using a piezoelectric phenomenon includes a piezoelectric micromachined ultrasonic transducer (PMUT). Moreover, an example of the detection element using a change in electrostatic capacitance includes a capacitive micromachined ultrasonic transducer (CMUT). Since the CMUT can detect photoacoustic waves in a wider frequency band, it is preferable as the detection element.

For acquisition of a high-resolution photoacoustic image, a plurality of detection elements is desirably arrayed in a two-dimensional manner or a three-dimensional manner to perform scanning. A reflective film such as a gold film can be provided on a surface of the probe so that light reflected by a subject or a surface of the support portion or light from a subject after scattering inside the subject returns to the subject again.

(Information Acquisition Unit)

An information acquisition unit in the present exemplary embodiment processes electric signals output by a measurement unit to acquire information about a subject. In other words, such an information acquisition unit can be referred to as a signal processing unit.

The information acquisition unit according to the present exemplary embodiment uses signals received by the measurement unit to generate data relating to optical property distribution information such as absorption coefficient distribution inside a subject. Generally, in a case where the absorption coefficient distribution inside the subject is to be calculated, initial sound pressure distribution inside the subject is calculated based on the electric signals output from the measurement unit, and light fluence inside the subject is considered. Accordingly, the absorption coefficient distribution is calculated. As for generation of the initial sound pressure distribution, for example, back projection based on time domain can be used.

(Display Unit)

The photoacoustic apparatus according to the present exemplary embodiment can include a display unit for displaying an image formed by the information acquisition unit. Typically, a display such as a liquid crystal display is used as the display unit.

(Subject and Optical Absorber)

A description of a subject and an optical absorber is given below. However, it will be appreciated that the subject and the optical absorber are not part of the photoacoustic apparatus of the present exemplary embodiment. Possible uses of the photoacoustic apparatus using a photoacoustic effect according to the present exemplary embodiment include image capturing of blood vessels, diagnosis of malignant tumor or vascular disease of a human being or animal, and chemotherapy follow-up. As mentioned previously, the subject may comprise an optical absorber that absorbs the irradiation light. This optical absorber has a relatively high absorption coefficient which depends on (i.e. is a function of) the wavelength of irradiating light. Particular examples of the optical absorber include water, fat, protein, oxyhemoglobin, and/or reduced hemoglobin.

Information about the subject includes an optical absorption coefficient and oxygen saturation.

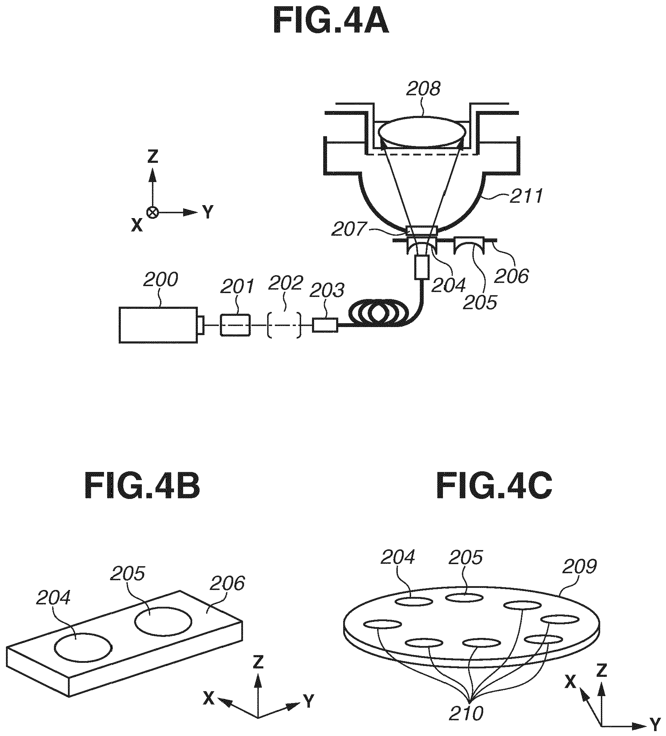

Fig. 4A, Fig. 4B, and Fig. 4C illustrate a configuration of a photoacoustic apparatus according to a second exemplary embodiment. This configuration is similar to the configuration of the first embodiment and therefore, for the sake of brevity, the below description focusses mainly on the differences between these two embodiments. That is, descriptions of the configurations and components which are similar to the first exemplary embodiment are omitted in the below description of the second embodiment.

The optical system adjustment unit in the first exemplary embodiment is integration of an optical system and a cup-shaped sensor. In the present exemplary embodiment, each of the optical system adjustment units 206 and 209 has a configuration in which a focal distance can be changed by moving a plurality of lenses 204 and 205 having different focal distances with respect to one cup-shaped sensor 211. For example, the position of a given adjustment unit 206, 209 relative to the cup-shaped sensor 211 may be configured such that the adjustment unit 206, 209 may be moved along a Y direction so as to change lenses 204, 205, and thereby change which lens 204, 205 directs light to the cup-shaped sensor 211.

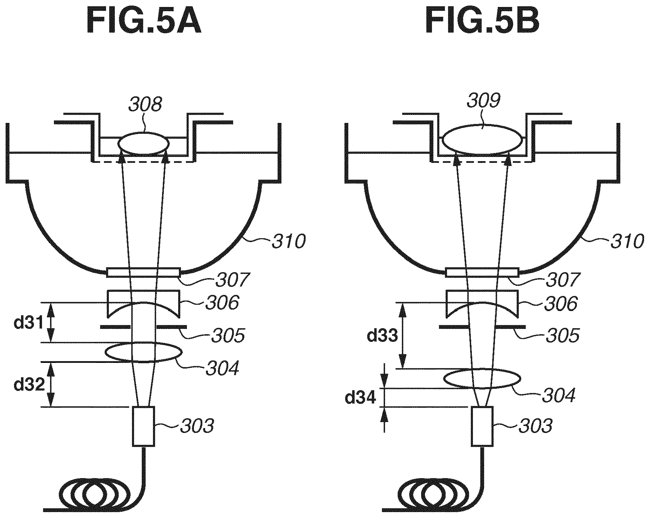

Figs. 5A and 5B illustrate a configuration of an optical system adjustment unit according to a third exemplary embodiment. This configuration is similar to the configuration of the first embodiment and therefore, for the sake of brevity, the below description focusses mainly on the differences between these the first and the third embodiments. That is, descriptions of the configurations and components which are similar to the first exemplary embodiment are omitted in the below description of the third embodiment. The optical system adjustment unit of the present exemplary embodiment includes one optical system including one cup-shaped sensor 310, a convex lens 304, a concave lens 306, and an aperture 305. The distance between the convex lens 304 and the concave lens 306 is changeable. A change in the distance between the lenses changes a focal distance of the optical system, thereby changing an irradiation range of illumination light to a subject 308,309.

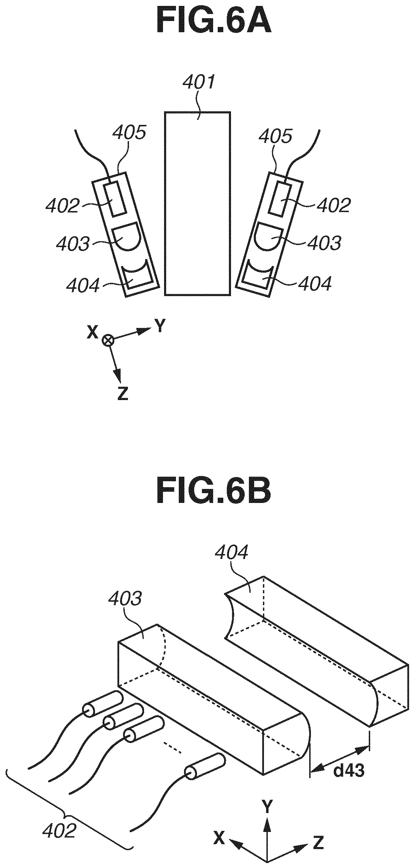

Figs. 6A and 6B illustrate a configuration of an optical system adjustment unit according to a fourth exemplary embodiment. This configuration is similar to the configuration of the first embodiment and therefore, for the sake of brevity, the below description focusses mainly on the differences between these the first and the fourth embodiments. That is, descriptions of the configurations and components which are similar to the first exemplary embodiment are omitted in the below description of the fourth embodiment. Light from a plurality of light sources 402 is formed into a sheet-shaped beam by using cylindrical lenses 403 and 404 for providing a desired size on an irradiation surface. Similar to the above-described third exemplary embodiment, a change in a relative distance between cylindrical lens 403 and cylindrical lens 404 can change an irradiation range of illumination light to a subject. That is, the area over which the sample is illuminated can be changed by changing the relative distance between the cylindrical lenses 403 and 404. Alternatively, a change in the relative distance between the light source 402 and the cylindrical lens 403 may also change the irradiation range of the illumination light on a subject.

[Examples]

A first example is described below with reference to Figs. 1A and 1B. This example describes the difference between the light quantity distribution of illumination light emitted onto the subject 111 using the arrangement of Fig. 1A and the light quantity distribution of illumination light emitted onto the subject 116 using the arrangement of Fig. 1B.

The subject 111 illustrated in Fig. 1A is, for example, the palm of a hand or the sole of a foot, and has an FOV range having a diameter of $\varphi$ 40 mm in a photoacoustic apparatus. The irradiation range of illumination light is preferably set with respect to the FOV range (which in this example has a diameter $\varphi$ of 40 mm) so that the entire area of the FOV is at least irradiated with the illumination light. Herein, the irradiation range of illumination light with respect to the FOV is increased by 10% of the FOV and has a diameter $\varphi$ of 44 mm. Quantitative definition of the irradiation range is from the position at which the energy density of light emitted to a subject becomes maximum to the position at which the energy density of light emitted to a subject becomes $1/e^2$ of the maximum energy density of light thereof.

On the other hand, the subject 116 illustrated in Fig. 1B is, for example, one finger, and an FOV with respect to the subject 116 is narrower than that with respect to the subject 111. Herein, the FOV range has a diameter $\varphi$ of 20 mm, and the illumination range has a diameter $\varphi$ of 22 mm.

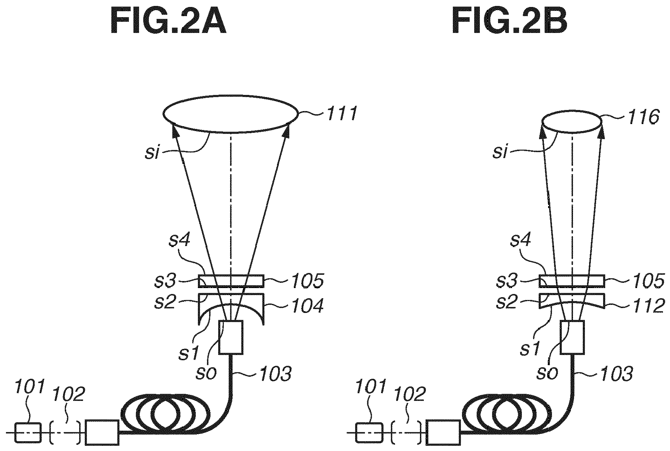

Fig. 2A is an enlarged view of one portion of Fig. 1A, whereas Fig. 2B is an enlarged view of one portion of Fig. 1B. Figs. 2A and 2B each mainly illustrate the arrangement of the optical components between an emission end of the optical fiber 103 and the subject 111, 116. Herein, the mesh member 110 and the waterproof member 109 illustrated in Figs. 1A and 1B are excluded for the sake of simplicity.

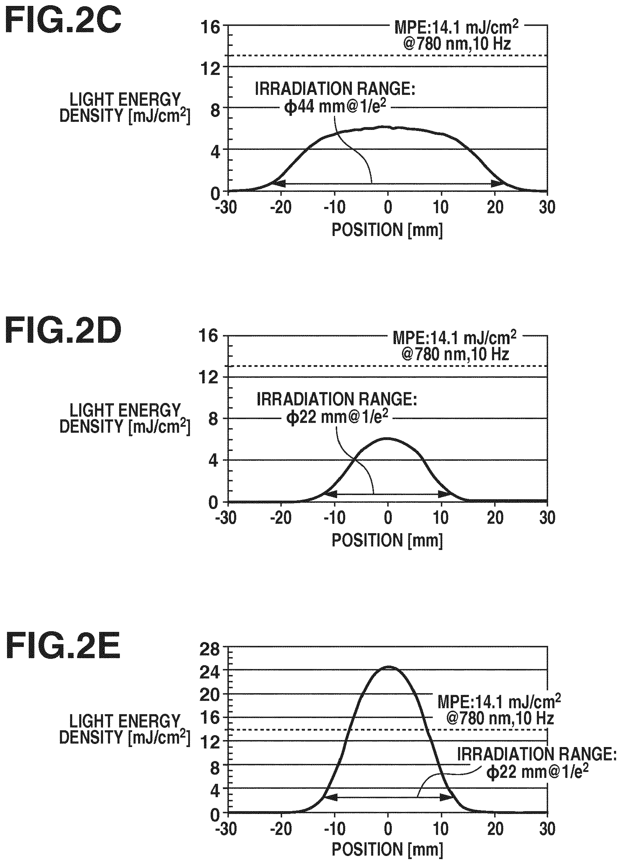

Light energy density distribution illustrated in Fig. 2C represents distribution at a surface of the subject 111 illustrated in Fig. 2A. In this example, a range (i.e. area) with a diameter $\varphi$ of 44 mm (@1/$e^2$) is illuminated by light and the FOV range has a diameter $\varphi$ of 40 mm. The maximum value of the light energy density is 6.1 mJ/cm$^2$, and the maximum permissible exposure (MPE) value with respect to the skin of human body is 14.1 mJ/cm$^2$ or less. Herein, the light quantity adjustment unit 101 adjusts transmittance of light from the light source unit 100 so that the maximum value of light energy density of irradiation light on a body surface of the subject 111 does not exceed the MPE value.

Similarly, light energy density distribution illustrated in Fig. 2D represents distribution at a surface of the subject 116 illustrated in Fig. 2B. A range having a diameter $\varphi$ of 22 mm (@1/$e^2$) is illuminated by light with respect to the FOV range having a diameter $\varphi$ of 20 mm. The maximum value of the light energy density is 6.1 mJ/cm$^2$, and an MPE value with respect to skin of human body is 14.1 mJ/cm$^2$ or less. Herein, the light quantity adjustment unit 101 adjusts transmittance of light from the light source unit 100 so that the maximum value of light energy density of irradiation light on a body surface of the subject 116 does not exceed the MPE value.

A change in the illumination range is changed by a change in a cup-shaped probe and changes in the concave lenses 104 and 112 having different focal distances.

Light energy density distribution illustrated in Fig. 2E represents the distribution that occurs in a case where the transmittance of the light quantity adjustment unit 101 is not changed when switching from the optical arrangement of Fig. 2A to the optical arrangement of Fig. 2B. However, in this case, the diameter of the illumination range is changed from $\varphi$ 44 mm to $\varphi$ 22 mm, but without changing a light quantity. This causes the maximum value of the light energy density to be 24.6 mJ/cm$^2$. Therefore, it will be appreciated that the maximum value of the light energy density exceeds the MPE value, and thus the human body cannot be irradiated with light at this rate. Accordingly, the light quantity adjustment unit 101 decreases the light quantity to adjust the maximum value of the light energy density to be the MPE value or less. A result of such adjustment is illustrated in Fig. 2D.

Tables 1 and 2 (shown below) respectively specify the calculated optical settings of components s0-si in Fig. 2A and Fig. 2B for providing the above-mentioned light energy density distributions illustrated in Figs. 2C, 2D, and 2E. These calculations were performed using LightTools (developed by Synopsys, Inc.).

The emission end of the optical fiber 103 has a diameter $\varphi$ of 10 mm and a NA (numerical aperture) of 0.22. The pulsed laser beam has a wavelength of 780 nm. A quantity of light energy at the optical fiber emission end is 93 mJ/pulse in each of Figs. 2C and 2E, and 23 mJ/pulse in Fig. 2D.

[Table 1]

Table 1: Optical Setting Value for Fig. 2A

| SURFACE # | RADIUS OF CURVATURE (mm) | THICKNESS (mm) | GLASS MATERIAL NAME | COMPONENT NAME |
|---|---|---|---|---|
| so | | 3.0 | | EMISSION END (OBJECT SURFACE)OF OPTICAL FIBER 103 |
| s1 | -11 | 2.5 | SYNTHETIC QUARTS | LENS 104: FOCAL DISTANCE = -23.9 mm |
| s2 | $\infty$ | 1.0 | | |
| s3 | $\infty$ | 3.0 | SYNTHETIC QUARTS | PARALLEL FLAT PLATE 105 |
| s4 | $\infty$ | 70.0 | WATER | |
| si | | | | SUBJECT 111 (IMAGE SURFACE) |

[Table 2]

| SURFACE # | RADIUS OF CURVATURE (mm) | THICKNESS (mm) | GLASS MATERIAL NAME | COMPONENT NAME |
|---|---|---|---|---|
| so | | 3.0 | | EMISSION END (OBJECT SURFACE)OF OPTICAL FIBER 103 |
| s1 | -40 | 2.5 | SYNTHETIC QUARTS | LENS 112: FOCAL DISTANCE = -87.0 mm |
| s2 | $\infty$ | 1.0 | | |
| s3 | $\infty$ | 3.0 | SYNTHETIC QUARTS | PARALLEL FLAT PLATE 105 |
| s4 | $\infty$ | 70.0 | WATER | |
| si | | | | SUBJECT 116 (IMAGE SURFACE) |

A second example is described below with reference to Fig. 4A. A plurality of lenses having different focal distances may be arrayed on a substrate in a one-dimensional manner or two-dimensional manner. In such a case, a lens can be selected by sliding the substrate to appropriately couple a desired lens to the subject and the output from the optical fiber or, more generally, the light emission unit. A plurality of lenses may be arranged along the circumference of a disk-shaped base. In such a case, a lens can be selected by rotating the disk-shaped base.

An inside of the cup-shaped sensor 211 is filled with water, and a parallel flat plate 207 (made of optical glass) for allowing illumination light from the optical fiber 203 to be transmitted inside the cup-shaped sensor 211 is attached to a bottom portion of the cup-shaped sensor 211. However, in other examples, such an optical component may not necessarily be a parallel flat plate, and instead a lens for controlling an irradiation range can be used, for example.

A third example is described below with reference to Fig. 5A and 5B. In the present example, one cup-shaped sensor 310 is installed with respect to one photoacoustic apparatus and one optical system. The optical system includes lenses 304 and 306, aperture 305, and parallel flat plate 307. In a similar manner to the above-mentioned second exemplary embodiment, the parallel flat plate allows illumination light to be transmitted inside the cup-shaped sensor 310 and is arranged below the cup-shaped sensor 310. Each of lens distances d31 and d33 and object distances d32 and d34 can be optionally changed. Preferably, these distances d31-d32 are changed according to the size of the FOV.

Fig. 5A illustrates lens arrangement if an FOV is narrow, whereas Fig. 5B illustrates lens arrangement if an FOV is wide. A position of the aperture 305 for removing unnecessary light is not limited to that illustrated in Fig. 5A or 5B. The aperture 305 can be positioned between the optical fiber 303 and the lens 304 or the lens 306 and the parallel flat plate 307.

A fourth example is described below with reference to Fig. 6A and Fig. 6B. In the present example, the photoacoustic apparatus is applied to a hand-held probe as illustrated in Figs. 6A and 6B. On each of both sides of a hand-held probe 401, a lens-barrel 405 in which a light source 402, and cylindrical lenses 403 and 404 are arranged is attached. A relative distance between the cylindrical lenses 403 and 404 can be optionally changed, and a change in the distance changes an irradiation range of light to be emitted to a subject from a light source unit.

According to the photoacoustic apparatus of each of the above-described exemplary embodiments, a quantity and an irradiation range of light to be emitted to a subject can be adjusted according to the size of a field of view.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A photoacoustic apparatus comprising:

   a light emission means configured to emit light to a subject;
   an ultrasonic wave probe configured to detect an ultrasonic wave generated from the subject irradiated with the light and output an electric signal;
   an information acquisition means configured to acquire information about the subject based on at least the electric signal;

an optical system adjustment means configured to adjust an area over which the subject is irradiated; and a light quantity adjustment means configured to adjust intensity of light to be emitted to the subject, wherein the optical system adjustment means is configured to change the irradiation range according to size of a field of view of the photoacoustic apparatus, and

wherein the light quantity adjustment means is configured to change the light quantity according to size of a field of view of the photoacoustic apparatus.

2. The photoacoustic apparatus according to claim 1, wherein the field of view defines an area from a position at which sensitivity for detection of the ultrasonic wave by the photoacoustic apparatus is a maximum value to a position at which the sensitivity is a half of the maximum value.

3. The photoacoustic apparatus according to claim 1 or 2, wherein the field of view is defined based on at least the size of a detection element, a position at which the detection element is arranged, and a characteristic of a response frequency of the detection element.

4. The photoacoustic apparatus according to any of claims 1 to 3, wherein the ultrasonic wave probe includes a cup-shaped support portion, and a plurality of detection elements that are arranged on the support portion and configured to detect an ultrasonic wave.

5. The photoacoustic apparatus according to claim 4,
wherein each of the detection elements have a circular surface that detects an ultrasonic wave, and
wherein the field of view is defined based on at least a radius of the circular surface that detects the ultrasonic wave, a radius of the cup-shaped support portion, and a maximum value of response frequency of the detection elements.

6. The photoacoustic apparatus according to any of claims 1 to 5, wherein the optical system adjustment means includes a plurality of lenses that each have a different focal distance, and is configured so that light from the light emission means is emitted to the subject through any of the plurality of lenses, and
wherein the lens through which the light from the light emission means passes differ according to the size of the field of view.

7. The photoacoustic apparatus according to any of claims 1 to 6, wherein the ultrasonic wave probe • is replaceable with a different ultrasonic wave probe that has a different field of view size.

8. The photoacoustic apparatus according to claim 6, wherein the optical system adjustment means is configured to move the position of at least one of lenses in the light emission means so as to change the irradiation range.

9. The photoacoustic apparatus according to any one of claims 1 to 8, wherein the light quantity adjustment means includes a $\lambda/2$ wavelength plate and a polarization beam splitter.

10. The photoacoustic apparatus according to claim 9, wherein the light quantity adjustment means further includes an optical absorption member capable of absorbing light reflected by the polarization beam splitter.

11. The photoacoustic apparatus according to any one of claims 1 to 10, wherein the light quantity adjustment means includes a plurality of optical filters having transmittances that differ from each other.

12. The photoacoustic apparatus according to claim 4, wherein the support portion is a hand-held type support portion.

EP 3 351 166 A1

# FIG.1A

# FIG.1B

11

**FIG.2A**

**FIG.2B**

**FIG.2C**

**FIG.2D**

**FIG.2E**

# FIG.3A

101

118    119

120

# FIG.3B

101

121

122

122

# FIG.4A

208

Z

X ⊗ → Y

211

207

204
206
205

200    201    202    203

# FIG.4B

205

204        206

Z
X ↙ → Y

# FIG.4C

204    205        209

210

X ↖ Z ↑ → Y

# FIG.5A

# FIG.5B

# FIG.6A

# FIG.6B

Europäisches
Patentamt

European
Patent Office

Office européen
des brevets

# EUROPEAN SEARCH REPORT

Application Number

EP 18 15 1048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/197343 A1 (MIYASATO TAKURO [JP]) 1 August 2013 (2013-08-01) * paragraph [0024] * * paragraph [0048] * * paragraph [0076] * * paragraph [0092] - paragraph [0094] * * paragraph [0096] - paragraph [0098] * * figure 1 * * figures 3A-C * | 1-12 | INV. A61B5/00 |
| A | WO 2011/070985 A1 (CANON KK [JP]; SATO AKIRA [JP]; SUDO YOSHIAKI [JP]) 16 June 2011 (2011-06-16) * paragraph [0054] * * figures 1,4,5 * | 1 | |
| A | EP 2 299 897 B1 (CANON KK [JP]) 30 March 2011 (2011-03-30) * paragraph [0185] - paragraph [0186] * | 1,2,6 | |
| A | US 6 102 857 A (KRUGER ROBERT A [US]) 15 August 2000 (2000-08-15) * column 8 - column 9 * * figures 8,9 * | 1,4,5 | TECHNICAL FIELDS SEARCHED (IPC) A61B G01N |
| A | VOLKER NEUSCHMELTING ET AL: "Performance of a Multispectral Optoacoustic Tomography (MSOT) System equipped with 2D vs. 3D Handheld Probes for Potential Clinical Translation", PHOTOACOUSTICS, vol. 4, no. 1, 1 March 2016 (2016-03-01), pages 1-10, XP055456257, ISSN: 2213-5979, DOI: 10.1016/j.pacs.2015.12.001 * section 2.2; page 2 * * figures 1A-B * | 1,4,7 | |

-/--

The present search report has been drawn up for all claims

| Place of search | Date of completion of the search | Examiner |
|---|---|---|
| The Hague | 6 March 2018 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 18 15 1048

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROBERT A. KRUGER ET AL: "Dedicated 3D photoacoustic breast imaging : Dedicated 3D photoacoustic breast imaging", MEDICAL PHYSICS., vol. 40, no. 11, 10 October 2013 (2013-10-10), page 113301, XP055457003, US ISSN: 0094-2405, DOI: 10.1118/1.4824317 * figures 2-3 * | 4 | |
| A | EP 2 946 723 A1 (CANON KK [JP]) 25 November 2015 (2015-11-25) * figures 2A-B * | 4,5 | |

TECHNICAL FIELDS
SEARCHED      (IPC)

The present search report has been drawn up for all claims

| Place of search | Date of completion of the search | Examiner |
|---|---|---|
| The Hague | 6 March 2018 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 18 15 1048

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2018

| Patent document cited in search report | | Publication date | Patent family member(s) | | Publication date |
|---|---|---|---|---|---|
| US 2013197343 | A1 | 01-08-2013 | CN | 104053402 A | 17-09-2014 |
| | | | EP | 2805676 A1 | 26-11-2014 |
| | | | JP | 5871958 B2 | 01-03-2016 |
| | | | JP | WO2013108375 A1 | 11-05-2015 |
| | | | RU | 2014133557 A | 10-03-2016 |
| | | | US | 2013197343 A1 | 01-08-2013 |
| | | | US | 2018028067 A1 | 01-02-2018 |
| | | | WO | 2013108375 A1 | 25-07-2013 |
| WO 2011070985 | A1 | 16-06-2011 | JP | 5538855 B2 | 02-07-2014 |
| | | | JP | 2011120795 A | 23-06-2011 |
| | | | US | 2013031982 A1 | 07-02-2013 |
| | | | WO | 2011070985 A1 | 16-06-2011 |
| EP 2299897 | B1 | 30-03-2011 | BR | PI0915675 A2 | 04-02-2014 |
| | | | BR | 122012009139 A2 | 14-07-2015 |
| | | | CN | 102083359 A | 01-06-2011 |
| | | | CN | 102940480 A | 27-02-2013 |
| | | | EP | 2299897 A1 | 30-03-2011 |
| | | | JP | 4829934 B2 | 07-12-2011 |
| | | | JP | 2010017426 A | 28-01-2010 |
| | | | RU | 2011105013 A | 20-08-2012 |
| | | | US | 2011112391 A1 | 12-05-2011 |
| | | | WO | 2010005109 A1 | 14-01-2010 |
| US 6102857 | A | 15-08-2000 | AT | 408374 T | 15-10-2008 |
| | | | AU | 725072 B2 | 05-10-2000 |
| | | | BR | 9712262 A | 25-01-2000 |
| | | | CA | 2187701 A1 | 04-04-1998 |
| | | | EP | 0942683 A1 | 22-09-1999 |
| | | | JP | 4341987 B2 | 14-10-2009 |
| | | | JP | 2001507952 A | 19-06-2001 |
| | | | US | 5713356 A | 03-02-1998 |
| | | | US | 6102857 A | 15-08-2000 |
| | | | US | 6292682 B1 | 18-09-2001 |
| | | | US | 2002035327 A1 | 21-03-2002 |
| | | | WO | 9814118 A1 | 09-04-1998 |
| EP 2946723 | A1 | 25-11-2015 | EP | 2946723 A1 | 25-11-2015 |
| | | | JP | 2015216979 A | 07-12-2015 |
| | | | US | 2015327768 A1 | 19-11-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2016112168 A